(19) Europäisches Patentamt

European Patent Office

Office européen des brevets

(11) **EP 0 982 070 B1**

(12) **EUROPÄISCHE PATENTSCHRIFT**

(45) Veröffentlichungstag und Bekanntmachung des Hinweises auf die Patenterteilung:
**29.10.2003 Patentblatt 2003/44**

(51) Int Cl.[7]: **B01J 23/36**, B01J 37/02, C08G 61/08

(21) Anmeldenummer: **99115168.9**

(22) Anmeldetag: **13.08.1999**

(54) **Rhenium-enthaltender Metathesekatalysator, Verfahren zu seiner Herstellung und seine Verwendung**

Metathesis catalyst containing rhenium, preparation and use thereof

Catalyseur de métathèse à base de rhénium, sa préparation et son utilisation

(84) Benannte Vertragsstaaten:
**AT BE CH CY DE DK ES FI FR GB GR IE IT LI LU MC NL PT SE**

(30) Priorität: **17.08.1998 DE 19837203**

(43) Veröffentlichungstag der Anmeldung:
**01.03.2000 Patentblatt 2000/09**

(73) Patentinhaber: **BASF AKTIENGESELLSCHAFT**
**67056 Ludwigshafen (DE)**

(72) Erfinder:
• **Schwab, Peter, Dr.**
**67098 Bad Dürkheim (DE)**
• **Breitscheidel, Boris, Dr.**
**67117 Limburgerhof (DE)**

• **Schulz, Ralf, Dr.**
**67346 Speyer (DE)**
• **Schulz, Michael, Dr.**
**67067 Ludwigshafen (DE)**
• **Müller, Ulrich, Dr.**
**67435 Neustadt (DE)**

(74) Vertreter: **Isenbruck, Günter, Dr. et al**
**Isenbruck, Bösl, Hörschler, Wichmann, Huhn,**
**Patentanwälte**
**Theodor-Heuss-Anlage 12**
**68165 Mannheim (DE)**

(56) Entgegenhaltungen:
EP-A- 0 438 134          US-A- 4 659 686
US-A- 4 795 734          US-A- 5 143 885

**Beschreibung**

Die Erfindung betrifft $Re_2O_7$-Metathesekatalysatoren, Verfahren zur Herstellung dieser Katalysatoren sowie deren Einsatz in Metathesereaktionen von Olefinen.

Für die Katalyse von Metathesereaktionen können sowohl homogene als auch heterogene Katalysatoren eingesetzt werden. Bei technischen Metatheseverfahren werden bevorzugt heterogene Katalysatoren eingesetzt, insbesondere $Re_2O_7$-haltige Trägerkatalysatoren. In der Patentliteratur sind eine Reihe von $Re_2O_7$-haltigen Trägerkatalysatoren sowie Verfahren zu ihrer Herstellung beschrieben. So wird beispielsweise in GB-A- 1 105 564 die Herstellung $Re_2O_7$-haltiger Trägerkatalysatoren mittels Verdampfen von $Re_2O_7$ in einem Inertgasstrom bei Temperaturen zwischen 150°C und 700°C und anschließendem Abscheiden des $Re_2O_7$ aus dem Inertgasstrom auf einen Träger beschrieben.

In US 4,795,734 werden $Re_2O_7$-haltige Trägerkatalysatoren beschrieben, die durch Porentränkung eines aluminiumoxidhaltigen Trägers mit einer Lösung einer Rheniumkomponente hergestellt werden, wobei der imprägnierte Träger nach der Zugabe der rheniumhaltigen Tränklösung für einen Zeitraum von mindestens 10 h bei Temperaturen zwischen 0°C und 80°C unter Bedingungen, bei denen das Lösungsmittel nicht verdampft, stehengelassen wird. Wird der imprägnierte Träger bei Temperaturen oberhalb von Raumtemperatur stehengelassen, so wird die Imprägnierung durchgeführt, indem der Träger und die Tränklösung bereits vor der Imprägnierung auf die entsprechende Temperatur erwärmt werden und diese Temperatur während der Imprägnierung gehalten wird. Nach dem Imprägnieren und Stehenlassen wird der getränkte Träger bei Temperaturen von 85°C bis 250°C getrocknet und durch Erhitzen auf Temperaturen von 400°C bis 1000°C aktiviert.

Die Nachteile der in der Patentliteratur beschriebenen $Re_2O_7$-haltigen Trägerkatalysatoren bezüglich des Einsatzes bei Metathesereaktionen liegen darin, daß diese Katalysatoren für eine wirtschaftliche Durchführung der Verfahren zu geringe katalytische Aktivitäten und Selektivitäten und vor allem zu kurze Zyklus- und Standzeiten besitzen.

Hohe katalytische Aktivitäten und Selektivitäten sowie insbesondere lange Zyklus- und Standzeiten werden mit Trägerkatalysatoren erfahrungsgemäß dann erzielt, wenn die Aktivkomponte eines Trägerkatalysators, im vorliegenden Fall $Re_2O_7$, in möglichst hochdisperser Form auf dem Träger vorliegt, d. h. die $Re_2O_7$-Partikel müssen möglichst klein und die Rheniumoberfläche muß möglichst groß sein, und wenn die Aktivkomponente in Form einer möglichst dünnen äußeren Schale über den Träger verteilt ist. Es bestand somit die Aufgabe, $Re_2O_7$-haltige Trägerkatalysatoren zu finden, bei denen die $Re_2O_7$-Komponente hochdispers in Form einer äußeren Schale über den Träger verteilt ist, und die dementsprechend hohe katalytische Aktivitäten und Selektivitäten sowie insbesondere lange Zyklus- und Standzeiten aufweisen.

Es wurde gefunden, daß mittels spezieller Präparationsverfahren $Re_2O_7$-haltige Trägerkatalysatoren hergestellt werden können, bei denen die $Re_2O_7$-Komponente in Form von $Re_2O_7$-Partikeln mit einem Durchmesser < 1 nm auf dem anorganischen Träger vorliegt, die Rheniumoberfläche, bestimmt mittels $N_2O$-Puls-Chemisorption, mindestens 0,4 m²/g beträgt und die $Re_2O_7$-Komponente über den Träger in Form einer äußeren Schale mit einer Dicke von maximal 1,5 mm verteilt ist.

Gegenstand der Erfindung ist darüber hinaus der Einsatz der so hergestellten Katalysatoren für Metathesereaktionen von Olefinen, insbesondere zur Umwandlung von $C_4$- und $C_5$-Schnitten, beispielsweise aus Steamcrackern, in wirtschaftlich interessante Produkte, wie beispielsweise zur Gewinnung von Propylen aus $C_4$-Olefinen.

Gegenstand der Erfindung ist insbesondere der Einsatz der erfindungsgemäßen Katalysatoren für die Herstellung von Propen durch Metathese von $C_4$-Olefinen, wie sie z.B. in DE-A-196 40 026, DE-A-197 40 895 und DE-A-197 46 040 beschrieben ist.

Die erfindungsgemäßen Katalysatoren enthalten $Re_2O_7$ als Aktivkomponente, das in hochdisperser Form auf einen Träger aufgebracht ist. Als Träger eignen sich beispielsweise Aktivkohlen, Siliciumcarbid, Aluminiumoxide, Siliciumdioxid, Titandioxid, Zirconiumdioxid, Zinkoxid, Magnesiumoxid oder deren Gemische, bevorzugt Aluminiumoxide, ganz besonders bevorzugt $\gamma$-$Al_2O_3$. Die Träger können beispielsweise in Form von Tabletten, Extrudaten oder Granulat mit Durchmessern von beispielsweise 3 mm bis 5 mm vorliegen. Der Gehalt an $Re_2O_7$ in den erfindungsgemäßen Katalysatoren liegt bei 1 bis 50 Gew.-%, bevorzugt 5 bis 20 Gew.-%, besonders bevorzugt 9 bis 11 Gew.-%.

Die erfindungsgemäßen $Re_2O_7$-haltigen Trägerkatalysatoren zeichnen sich vor allem dadurch aus, daß die $Re_2O_7$-Komponente in Form von $Re_2O_7$-Partikeln mit einem Durchmesser kleiner als 1 nm, bevorzugt kleiner als 0,7 nm, auf dem Träger vorliegt, daß die Rheniumoberfläche - bestimmt mittels $N_2O$-Puls-Chemisorption - mindestens 0,4 m²/g, bevorzugt mindestens 0,5 m²/g beträgt, und daß die $Re_2O_7$-Komponente über den Träger in Form einer äußeren Schale mit einer Dicke von maximal 1,5 mm, bevorzugt maximal 1,0 mm, verteilt ist.

Die Größe der $Re_2O_7$-Partikeln wird mittels Transmissionselektronenmikroskopie bestimmt.

Die Pheniumoberfläche der erfindungsgemäßen Katalysatoren wird bestimmt mit Hilfe der $N_2O$-Puls-Chemisorption. Die Methode ist im folgenden näher erläutert:

**Gerät:**

PulseChemSorb 2705 der Firma Micromeritics.

**Probenvorbehandlung:**

Etwa 1,1 g Katalysator werden in einen Quarz-U-Rohrreaktor mit verbreitertem Probenteil ($d_a$ entspricht 11 mm) gegeben. Die Probe wird im Strom von 5% $H_2$/Ar (30 ml/min) mit einer Aufheizrate von 80 K/min von Raumtemperatur bis 400°C aufgeheizt. Es folgt eine einstündige Reduktion mit Wasserstoff 5.0 (30 ml/min) bei 400°C. Die Probe wird danach 30 min in einem He-Strom mit 30 ml/min bei 400°C eluiert und unter diesem Gas auf 70°C abgekühlt.

**Durchführung der Messung**

Zur Messung der relativen $N_2$O-Chemisorption werden mittels einer Dosierschleife (Volumen : 1000 µl) $N_2$O-Pulse in einen Heliumstrom von 30 ml/min dosiert und bei 70°C durch die Probe geleitet. Es wird solange gepulst, bis vier gleiche Pulse (jeweils gleiche $N_2$O-Menge) hintereinander detektiert werden. Die Analyse der verbrauchten Menge an $N_2$O beziehungsweise des entstandenen $N_2$ erfolgt an einer dem Reaktor nachgeschalteten 0,5 m langen chromatographischen Trennsäule mit Porapak-N®, welche mittels eines Wärmeleitfähigkeitsdetektors aufgezeichnet wird.

**Auswertung der Messung:**

1. Aus der Konstanz von vier Pulsen mit dem jeweiligen Flächeninhalt $PF^i$ wird ein Mittelwert der Pulsfläche ($MPE$) in willkürlichen Einheiten [w.F.] ermittelt. Bei insgesamt $n$ Pulsen also

$$(1) \qquad MPF = \sum_{i=n-3}^{n} PF^i / 4$$

Aus diesem Wert $MPF$ können mit Hilfe des Referenzvolumens $RV$ die Pulsflächen in µl umgerechnet werden.

$$(2) \qquad x MPF[\text{w.E.}] = y RV[\mu l]$$

2.1. Die Menge an adsorbiertem Gas $AdG$ wird aus dem Flächeninhalt der n Einzelpulse $PF^i$ wie folgt berechnet:

2.

$$(3) \qquad AdG[\text{w.E}] = n^* MPF - \sum_{i=1}^{n} PF^i = (n-4)^* MPF - \sum_{i=1}^{n \, 4} PF^i$$

3. Mit Hilfe von (2) wird die Menge an adsorbiertem Gas $AdG$ in µl berechnet.

4. Die adsorbierte Gasmenge $AdG$ [µl] wird auf Standartbedingungen $AdG^s$ [µl] umgerechnet.

$$(4) \qquad AdG^s = AdG * \frac{273 * p^m}{760 * T^m}$$

$p^m$ : Druck [Torr]; $T^m$ : Adsorbtionstemperatur [K]
$p^m$ und $T^m$ werden vor der Messung als Parameter eingegeben.

5. Aus $AdG^s$ wird durch die Normierung auf das Probengewicht $G$ die erste Meßgröße, die **spezifische adsorbierte Gasmenge** $V_m$ **[cm³/g] berechnet.**

$$(5) \qquad V_m[\text{cm}^3/\text{g}] = \frac{AdG^s[\mu l]}{1000 * G[g]}$$

6. Berechnung von $V_m$ in [µmol/g]

$$(6) \qquad V_m[\mu\text{mol/g}] = 44{,}940 * V_m[\text{cm}^3/\text{g}]$$

für $N_2O$ als Adsorptiv

7. Berechnung der spezifischen Rheniumoberfläche $S^m[\text{m}^2/\text{g}^{Kat}]$.

$$(7) \qquad S^m[m^2/g^{Kat}] = \frac{V_m[\mu\text{mol/l}] * S * N_L * 10^{-6}}{K}$$

$S$:     Stöchiometriefaktor (angenommen Re = 2)
$K$:     Anzahl der Metallatome pro m² (für Re = 1,54 x 10¹⁹m²)
$N_L$:     Avogadrozahl (6,022052 x 10²³ mol⁻¹)

Die Herstellung der erfindungsgemäßen Katalysatoren erfolgt mit Hilfe zweier spezieller Präparationsverfahren:

Verfahren A:

Bei Verfahren A werden die erfindungsgemäßen Katalysatoren hergestellt durch Porentränkung mit einer rheniumhaltigen Lösung, wobei die Tränklösung vor der Tränkung auf eine Temperatur von 40°C bis 80°C, bevorzugt 50°C bis 70°C, erwärmt wird. Wichtig hinsichtlich der Eigenschaften der erfindungsgemäßen Katalysatoren ist, daß auschließlich die Tränklösung auf Temperaturen von 40 °C bis 80 °C erwärmt wird, während der Träger bei Raumtemperatur gehalten wird. Nach der Aufbringung der Tränklösung auf den Träger läßt man die Tränklösung noch 1 bis 30 Minuten, bevorzugt 5 bis 20 Minuten, auf den Träger einwirken, bevor das Lösungsmittel mittels Trocknung entfernt wird. Die Trocknung wird bei Temperaturen von 80°C bis 170°C, bevorzugt 100 bis 150°C über einen Zeitraum von 1 bis 48 h, bevorzugt 12 bis 24 h durchgeführt. Die Trocknung kann ruhend oder bewegt erfolgen.
Als Lösungsmittel wird bevorzugt Wasser verwendet. Als Rhenium-Precursor für diese Präparationsvariante eignen sich beispielsweise Ammoniumperrhenat, Alkaliperrhenate, wässrige Perrheniumsäurelösung und Rhenium (VII)oxid, bevorzugt Ammoniumperrhenat.

Verfahren B:

Bei Verfahren B wird der Träger in einer drehbaren Tränktrommel vorgelegt, die mit 2 bis 100 Umdrehungen pro Minute, bevorzugt 5 bis 20 Umdrehungen pro Minute, gedreht wird. Der bewegte Träger wird mit einer rheniumhaltigen Lösung besprüht, wobei die Lösungsmenge dem Porenvolumen des vorgelegten Trägers entspricht und die Lösungsmenge innerhalb von 1 bis 120 Minuten, bevorzugt 5 bis 60 Minuten, besonders bevorzugt 10 bis 30 Minuten, aufgesprüht wird. Nach der Aufbringung der Tränklösung auf den Träger läßt man die Tränklösung noch 1 bis maximal 30 Minuten, bevorzugt 5 bis 20 Minuten, auf den bewegten Träger einwirken, bevor das Lösungsmittel mittels Trocknung entfernt wird. Die Trocknung wird bei Temperaturen von 80°C bis 170°C, bevorzugt 100 bis 150°C über einen Zeitraum von 1 bis 48 h, bevorzugt 12 bis 24 h durchgeführt. Die Trocknung kann ruhend oder bewegt erfolgen.
Als Lösungsmittel wird bevorzugt Wasser verwendet. Als Rhenium-Precursor für diese Präparationsvariante eignen sich beispielsweise Ammoniumperrhenat, Alkaliperrhenate, wässrige Perrheniumsäurelösung und Rhenium (VII)oxid, bevorzugt wässrige Perrheniumsäurelösung.

Die erfindungsgemäßen Katalysatoren werden vor Gebrauch in situ aktiviert durch Kalzinierung bei Temperaturen von 400 bis 700°C, bevorzugt 500 bis 600°C.

Die erfindungsgemäßen Katalysatoren zeichnen sich gegenüber den literaturbekannten $Re_2O_7$-haltigen Trägerkatalysatoren bei Metathesereaktionen durch hohe katalytische Aktivitäten und Selektivitäten aus. Die erfindungsgemäßen Katalysatoren zeichnen sich vor allem durch Zykluszeiten von mindestens 10 Tagen aus (unter einem Zyklus wird hier die Zeitspanne verstanden, in der die katalytische Aktivität des Katalysators auf die Hälfte der Anfangsaktivität des Katalysators zu Beginn des Zyklus abgefallen ist) und dadurch, daß nach der Beendigung eines Zyklus die katalytische Aktivität durch Kalzinierung des gebrauchten Katalysators bei Temperaturen von 400-600°C im Luftstrom und Abkühlen unter Inertgas vollständig, mindestens jedoch auf ein Niveau von 99 % bezüglich der Anfangsaktivität des Frischkatalysators wiederhergestellt werden kann. Darüber hinaus lassen sich die erfindungsgemäßen Katalysatoren mindestens vierzigmal regenerieren, so daß sehr hohe Standzeiten von mindestens einem Jahr (Standzeit: Zykluszeit x Anzahl der Zyklen) resultieren, die eine wirtschaftliche Durchführung von Metatheseverfahren erst ermöglichen.

Die Erfindung wird anhand der folgenden Beispiele näher erläutert.

**Herstellung Katalysator A:**

150 g eines $\gamma$-$Al_2O_3$-Trägers in Form von 4 mm-Extrudaten mit einer Oberfläche von 228 m$^2$/g und einer Porosität von 0,66 cm$^3$/g werden mit einer wässrigen Ammoniumperrhenatlösung, die durch Auflösen von 18,5 g Ammoniumperrhenat in 99 ml Wasser hergestellt und auf 60°C erwärmt wurde, getränkt. Nach der Aufbringung der Tränklösung auf den Träger läßt man die Tränklösung noch 15 Minuten auf den Träger einwirken. Anschließend wird der getränkte Träger sofort 16 h bei 120°C getrocknet. Der so erhaltene Katalysator enthält 10 Gew.-% $Re_2O_7$. Die Größe der $Re_2O_7$-Partikel auf der Trägeroberfläche, die Rheniumoberfläche und die Verteilung der $Re_2O_7$-Komponente über den Träger sind in Tabelle 1 zusammengestellt.

**Herstellung Katalysator B:**

150 g eines $\gamma$-$Al_2O_3$-Trägers in From von 4 mm-Extrudaten mit einer Oberfläche von 228 m$^2$/g und einer Porosität von 0,66 cm$^3$ werden in einer Tränktrommel vorgelegt, die mit einer Geschwindigkeit von 8 Umdrehungen pro Minute gedreht wird. Auf den bewegten Träger wird innerhalb von 25 Minuten 99 ml einer wässrigen Perrheniumsäurelösung aufgesprüht. Anschließend läßt man die Tränklösung noch 5 Minuten auf den bewegten Träger einwirken. Der getränkte Träger wird dann sofort 16 h bei 120°C getrocknet. Der so erhaltene Katalysator enthält 10 Gew.-% $Re_2O_7$. Die Größe der $Re_2O_7$-Partikel auf der Trägeroberfläche, die Rheniumoberfläche und die Verteilung der $Re_2O_7$-Komponente über den Träger sind in Tabelle 1 zusammengestellt.

**Herstellung Katalysator C (Vergleichskatalysator):**

Aus US 4 795 734 wurde der Katalysator aus Beispiel 4 (Spalte 4, Zeilen 43 -60) nachgestellt. Allerdings wurde die Präparation wegen der Vergleichbarkeit mit den erfindungsgemäßen Katalysatoren A und B ohne die Aktivierung bei 550 °C über 3 h und das Spülen mit Stickstoff (Zeilen 56 - 60) durchgeführt. Die Größe der $Re_2O_7$-Partikel auf der Trägeroberfläche, die Rheniumoberfläche und die Verteilung der $Re_2O_7$-Komponente über den Träger sind in Tabelle 1 zusammengestellt.

Tabelle 1

| Katalysator | Re-Oberfläche m$^2$/g | $Re_2O_7$-Partikel-größen nm | Re-Verteilung über den Träger |
|---|---|---|---|
| Katalysator A | 0,63 | 0,5 | 1,5 mm dicke äußere Schale |
| Katalysator B | 0,56 | 0,6 | 1,0 mm dicke äußere Schale |
| Katalysator C (Vergleichskatalysator) | 0,29 | 1,1 | homogen |

**Beispiel 1a: Metathese eines n-Buten-Gemisches an Katalysator A**

Raffinat II (40 % 1-Buten, 45 % cis/trans-2-Buten) wurde bei einer Reaktionstemperatur von 60°C, einem Druck von 10 bar und einer Katalysatorbelastung von 1000 g/l*h kontinuierlich über einen mit Katalysator A bestückten Rohrreaktor geleitet. Der Katalysator wurde vor Gebrauch in situ durch Kalzinierung bei einer Temperatur von 550 °C aktiviert. Der Reaktionsaustrag wurde gaschromatographisch online untersucht. Der Umsatz bezüglich 1-Buten lag anfangs bei 80% und fiel innerhalb von 15 Tagen auf 40%, d.h. die Zykluszeit betrug 15 Tage. Die Propen-Selektivität lag über die gesamte Zyklusdauer bei 90%. Der Katalysator wurde nach Beendigung des Zyklus durch Kalzinierung im Luftstrom bei 550°C und anschließendes Abkühlen unter Argon regeneriert. Zu Beginn des zweiten Zyklus lag der Umsatz bezüglich 1-Buten wieder bei 80 %, d.h. das Aktivitätsniveau des Frischkatalysators zu Beginn des ersten Zyklus wurde zu 100% wieder erreicht; die Propen-Selektivität lag auch über den gesamten zweiten Zyklus bei 90%. Insgesamt konnte der Katalysator A fünfzigmal regeneriert werden, bevor die Anfangsaktivität das Niveau von mindestens 99 % der Anfangsaktivität des Frischkatalysators nicht mehr erreichte: d.h. die Standzeit betrug 50 x 15 = 750 Tage.

**Beispiel 1b: Ethenolyse von 2-Penten an Katalysator A**

2-Penten wurde bei einer Temperatur von 40°C und einem Druck von 40 bar unter Zudosierung von Ethen im Molverhältnis C5/C2 von 1:1 bei einer Katalysatorbelastung von 1000 g/l x h kontinuierlich über einen mit Katalysator A bestückten Rohrreaktor geleitet. Der Katalysator wurde vor Gebrauch in situ durch Kalzinierung bei einer Temperatur von 550 °C aktiviert. Der Reaktoraustrag wurde gaschromatographisch online untersucht. Der Umsatz bezüglich des 2-Penten lag anfangs bei 86 % und fiel innerhalb von 14 Tagen auf 43 %, d.h. die Zykluszeit betrug 14 Tage. Die Propen-Selektivität lag über die gesamte Zyklusdauer bei 98 %. Der Katalysator wurde nach Beendigung des Zyklus durch Kalzinierung im Luftstrom bei 550°C und anschließendes Abkühlen unter Argon regeneriert. Zu Beginn des zweiten Zyklus lag der Umsatz bezüglich des 2-Penten wieder bei 86 %, d.h. das Aktivitätsniveau des Frischkatalysators zu Beginn des ersten Zyklus wurde zu 100% wieder erreicht; die Propen-Selektivität lag auch über den gesamten zweiten Zyklus bei 98 %. Insgesamt konnte der Katalysator A siebenundvierzigmal regeneriert werden, bevor die Anfangsaktivität das Niveau von mindestens 99 % der Anfangsaktivität des Frischkatalysators nicht mehr erreichte; d.h. die Standzeit betrug 47 x 15 = 705 Tage.

**Beispiel 2a: Metathese eines n-Buten-Gemisches an Katalysator B**

Raffinat II (40 % 1-Buten, 45 % cis/trans-2-Buten) wurde bei einer Reaktionstemperatur von 60°C, einem Druck von 10 bar und einer Katalysatorbelastung von 1000 g/l*h kontinuierlich über einen mit Katalysator B bestückten Rohrreaktor geleitet. Der Katalysator wurde vor Gebrauch in situ durch Kalzinierung bei einer Temperatur von 550 °C aktiviert. Der Reaktionsaustrag wurde gaschromatographisch online untersucht. Der Umsatz bezüglich 1-Buten lag anfangs bei 80 % und fiel innerhalb von 15 Tagen auf 40 %, d.h. die Zykluszeit betrug 15 Tage. Die Propen-Selektivität lag über die gesamte Zyklusdauer bei 90 %. Der Katalysator wurde nach Beendigung des Zyklus durch Kalzinierung im Luftstrom bei 550°C und anschließendes Abkühlen unter Argon regeneriert. Zu Beginn des zweiten Zyklus lag der Umsatz bezüglich 1-Buten wieder bei 80 %, d.h. das Aktivitätsniveau des Frischkatalysators zu Beginn des ersten Zyklus wurde zu 100% wieder erreicht; die Propen-Selektivität lag auch über den gesamten zweiten Zyklus bei 90 %. Insgesamt konnte der Katalysator B achtundvierzigmal regeneriert werden, bevor die Anfangsaktivität das Niveau von mindestens 99 % der Anfangsaktivität des Frischkatalysators nicht mehr erreichte: d.h. die Standzeit betrug 48 x 15 = 720 Tage.

**Beispiel 2b: Ethenolyse von 2-Penten an Katalysator B**

2-Penten wurde bei einer Temperatur von 40°C und einem Druck von 40 bar unter Zudosierung von Ethen im Molverhältnis C5/C2 von 1:1 bei einer Katalysatorbelastung von 1000 g/l x h kontinuierlich über einen mit Katalysator B bestückten Rohrreaktor geleitet. Der Katalysator wurde vor Gebrauch in situ durch Kalzinierung bei einer Temperatur von 550 °C aktiviert. Der Reaktoraustrag wurde gaschromatographisch online unterucht. Der Umsatz bezüglich 2-Penten lag anfangs bei 87 % und fiel innerhalb von 14 Tagen auf 44 %, d.h. die Zykluszeit betrug 14 Tage. Die Propen-Selektivität lag über die gesamte Zyklusdauer bei 97 %. Der Katalysator wurde nach Beendigung des Zyklus durch Kalzinierung im Luftstrom bei 550°C und anschließendes Abkühlen unter Argon regeneriert. Zu Beginn des zweiten Zyklus lag der Umsatz bezüglich 2-Penten wieder bei 87 %, d.h. das Aktivitätsniveau des Frischkatalysators zu Beginn des ersten Zyklus wurde zu 100% wieder erreicht; die Propen-Selektivität lag auch über den gesamten zweiten Zyklus bei 97 %. Insgesamt konnte der Katalysator B sechsundvierzigmal regeneriert werden, bevor die Anfangsaktivität das Niveau von mindestens 99 % der Anfangsaktivität des Frischkatalysators nicht mehr erreichte; d.h. die Standzeit betrug 46 x 15 = 690 Tage.

**Beispiel 3a (Vergleichsbeispiel):**

**Metathese eines n-Buten-Gemisches an Katalysator C (Vergleichskatalysator)**

Raffinat II (40 % 1-Buten, 45 % cis/trans-2-Buten) wurde bei einer Reaktionstemperatur von 60°C, einem Druck von 10 bar und einer Katalysatorbelastung von 1000 g/l*h kontinuierlich über einen mit Katalysator C bestückten Rohrreaktor geleitet. Der Katalysator wurde vor Gebrauch in situ durch Kalzinierung bei einer Temperatur von 550 °C aktiviert. Der Reaktionsaustrag wurde gaschromatographisch online analysiert. Der Umsatz bezüglich 1-Buten lag anfangs bei 78 % und fiel innerhalb von 9 Tagen auf 39 %, d.h. die Zykluszeit betrug nur 9 Tage. Die Propen-Selektivität lag über die gesamte Zyklusdauer bei 86 %. Der Katalysator wurde nach Beendigung des Zyklus durch Kalzinierung im Luftstrom bei 550°C und anschließendes Abkühlen unter Argon regeneriert. Zu Beginn des zweiten Zyklus lag der Umsatz bezüglich 1-Buten wieder bei 78 %, d.h. das Aktivitätsniveau des Frischkatalysators zu Beginn des ersten Zyklus wurde zu 100 % wieder erreicht; die Propen-Selektivität lag auch über den gesamten zweiten Zyklus bei 86 %. Insgesamt konnte der Katalysator C vierunddreißigmal regeneriert werden, bevor die Anfangsaktivität das Niveau von mindestens 99 % der Anfangsaktivität des Frischkatalysators nicht mehr erreichte: die Standzeit betrug somit nur 34 x 9 = 306 Tage.

**Beispiel 3b (Vergleichsbeispiel):**

**Ethenolyse von 2-Penten an Katalysator C (Vergleichskatalysator)**

2-Penten wurde bei einer Temperatur von 40°C und einem Druck von 40 bar unter Zudosierung von Ethen im Molverhältnis C5/C2 von 1:1 bei einer Katalysatorbelastung von 1000 g/l x h kontinuierlich über einen mit Katalysator C bestückten Rohrreaktor geleitet. Der Katalysator wurde vor Gebrauch in situ durch Kalzinierung bei einer Temperatur von 550 °C aktiviert. Der Reaktoraustrag wurde gaschromatographisch online untersucht. Der Umsatz bezüglich 2-Penten lag anfangs bei 80 % und fiel innerhalb von 8 Tagen auf 40 %, d.h. die Zykluszeit betrug nur 8 Tage. Die Propen-Selektivität lag über die gesamte Zyklusdauer bei 95 %. Der Katalysator wurde nach Beendigung des Zyklus durch Kalzinierung im Luftstrom bei 550°C und anschließendes Abkühlen unter Argon regeneriert. Zu Beginn des zweiten Zyklus lag der Umsatz bezüglich 2-Penten wieder bei 80 %, d.h. das Aktivitätsniveau des Frischkatalysators zu Beginn des ersten Zyklus wurde zu 100 % wieder erreicht; die Propen-Selektivität lag auch über den gesamten zweiten Zyklus bei 95 %. Insgesamt konnte der Katalysator C zweiunddreißigmal regeneriert werden, bevor die Anfangsaktivität das Niveau von mindestens 99 % der Anfangsaktivität des Frischkatalysators nicht mehr erreichte; d.h. die Standzeit betrug nur 32 x 8 = 256 Tage.

**Patentansprüche**

1. Katalysator, enthaltend $Re_2O_7$ auf einem anorganischen Träger, **dadurch gekennzeichnet, daß** die $Re_2O_7$-Komponente in Form von $Re_2O_7$-Partikeln mit einem Durchmesser < 1 nm auf dem Träger vorliegt, die Rheniumoberfläche, bestimmt mittels $N_2O$-Puls-Chemisorption, mindestens 0,4 $m^2$/g beträgt und die $Re_2O_7$-Komponente über den Träger in Form einer äußeren Schale mit einer Dicke von maximal 1,5 mm verteilt ist.

2. Katalysator nach Anspruch 1, **dadurch gekennzeichnet, daß** die $Re_2O_7$-Komponente in Form von $Re_2O_7$-Partikeln mit einem Durchmesser < 0,7 nm auf dem Träger vorliegt, die Rheniumoberfläche, bestimmt mittels $N_2O$-Puls-Chemisorption, mindestens 0,5 $m^2$/g beträgt und die $Re_2O_7$-Komponente über den Träger in Form einer äußeren Schale mit einer Dicke von maximal 1,0 mm verteilt ist.

3. Katalysator nach Anspruch 1 oder 2, **dadurch gekennzeichnet, daß** der Träger ausgewählt ist aus Aktivkohlen, Siliziumcarbid, Aluminiumoxiden, Siliziumdioxid, Titandioxid, Zirkoniumdioxid, Zinkoxid, Magnesiumoxid und Gemischen davon.

4. Katalysator nach Anspruch 3, **dadurch gekennzeichnet, daß** der Träger γ-Aluminiumoxid ist.

5. Katalysator nach einem der Ansprüche 1 bis 4, **dadurch gekennzeichnet, daß** der $Re_2O_7$-Gehalt, bezogen auf den Katalysator, 9 bis 11 Gew.-% beträgt.

6. Verfahren zur Herstellung von Katalysatoren nach einem der Ansprüche 1 bis 5 durch Porentränkung eines Trägers mit einer rheniumhaltigen Tränklösung und Trocknung bei 80°C bis 170°C über 1 bis 48 h, **dadurch gekennzeich-**

**net, daß** ausschließlich die Tränklösung vor der Tränkung auf eine Temperatur von 40 bis 80°C erwärmt wird und man die Tränklösung nach der Tränkung noch 1 bis 30 Minuten auf den Träger einwirken läßt.

7. Verfahren zur Herstellung von Katalysatoren nach einem der Ansprüche 1 bis 5, **dadurch gekennzeichnet, daß** der Träger in einer drehbaren Tränktrommel vorgelegt wird, die mit 2 bis 100 Umdrehungen pro Minute gedreht wird, mit einer rheniumhaltigen Tränklösung besprüht wird, wobei die Lösungsmenge dem Porenvolumen des vorgelegten Trägers entspricht, die Lösungsmenge innerhalb von 1 bis 120 Minuten aufgesprüht wird, und man die Tränklösung nach der Tränkung noch 1 bis 30 Minuten auf den Träger einwirken läßt, und der imprägnierte Träger bei 80°C bis 170°C über 1 bis 48 h getrocknet wird.

8. Verwendung von Katalysatoren nach einem der Ansprüche 1 bis 5 als Metathesekatalysatoren.

9. Verwendung der Katalysatoren nach einem der Ansprüche 1 bis 5 zur Herstellung von Propen durch Metathese von $C_4$-Olefinen.

**Claims**

1. A catalyst containing $Re_2O_7$ on an inorganic support, wherein the $Re_2O_7$ component is on the support in the form of $Re_2O_7$ particles having a diameter of < 1 nm, the rhenium surface area, determined by $N_2O$ pulse chemisorption, is at least 0.4 $m^2/g$, and the $Re_2O_7$ component is distributed over the support in the form of an outer shell having a maximum thickness of 1.5 mm.

2. A catalyst as claimed in claim 1, wherein the $Re_2O_7$ component is on the support in the form of $Re_2O_7$ particles having a diameter of < 0.7 nm, the rhenium surface area, determined by $N_2O$ pulse chemisorption, is at least 0.5 $m^2/g$, and the $Re_2O_7$ component is distributed over the support in the form of an outer shell having a maximum thickness of 1.0 mm.

3. A catalyst as claimed in claim 1 or 2, wherein the support is selected from activated carbons, silicon carbide, aluminum oxides, silicon dioxide, titanium dioxide, zirconium dioxide, zinc oxide, magnesium oxide and mixtures thereof.

4. A catalyst as claimed in claim 3, wherein the support is $\gamma$-aluminum oxide.

5. A catalyst as claimed in any one of claims 1 to 4, wherein the $Re_2O_7$ content, based on the catalyst, is from 9 to 11% by weight.

6. A process for the preparation of a catalyst as claimed in any one of claims 1 to 5 by impregnating the pores of a support with a rhenium-containing impregnation solution and drying the support for from 1 to 48 hours at from 80°C to 170°C, which comprises heating exclusively the impregnation solution to from 40 to 80°C before the impregnation, and allowing the impregnation solution to act on the support for a further 1 to 30 minutes after the impregnation.

7. A process for the preparation of a catalyst as claimed in any one of claims 1 to 5, wherein the support is introduced into a revolvable impregnation drum, which is revolved at from 2 to 100 revolutions per minute, and sprayed with a rhenium-containing impregnation solution, where the amount of solution corresponds to the pore volume of the introduced support, the amount of solution is sprayed on over the course of 1 to 120 minutes, and the impregnation solution is allowed to act on the support for a further 1 to 30 minutes after the impregnation, and the impregnated support is dried at from 80°C to 170°C for from 1 to 48 hours.

8. The use of a catalyst as claimed in any one of claims 1 to 5 as a metathesis catalyst.

9. The use of a catalyst as claimed in any one of claims 1 to 5 for the preparation of propene by the metathesis of $C_4$-olefins.

**Revendications**

1. Catalyseur, contenant $Re_2O_7$ sur un support inorganique, **caractérisé en ce que** le composant $Re_2O_7$ est présent sur le support sous forme de particules $Re_2O_7$ avec un diamètre < 1 nm, la surface du rhénium, déterminée par chimisorption pulsée du $N_2O$, s'élève à au moins 0,4 $m^2$/g et le composant $Re_2O_7$ est distribué sur le support sous la forme d'une enveloppe extérieure d'épaisseur maximale de 1,5 mm.

2. Catalyseur selon la revendication 1, **caractérisé en ce que** le composant $Re_2O_7$ est présent sous forme de particules $Re_2O_7$ avec un diamètre < 0,7 nm sur le support, la surface du rhénium, déterminée par chimisorption pulsée du $N_2O$, s'élève à au moins 0,5 $m^2$/g et le composant $Re_2O_7$ est distribué sur le support sous forme d'une enveloppe extérieure d'épaisseur maximale de 1,0 mm.

3. Catalyseur selon la revendication 1 ou 2, **caractérisé en ce que** le support est choisi parmi le charbon actif, le carbure de silicium, l'oxyde d'aluminium, le dioxyde de silicium, le dioxyde de titane, le dioxyde de zirconium, l'oxyde de zinc, l'oxyde de magnésium et leurs mélanges.

4. Catalyseur selon la revendication 3, **caractérisé en ce que** le support est le γ-oxyde d'aluminium.

5. Catalyseur selon l'une des revendications 1 à 4, **caractérisé en ce que** la quantité de $Re_2O_7$, recouvrant le catalyseur, représente 9 à 11 % en poids.

6. Procédé de fabrication de catalyseurs selon l'une des revendications 1 à 5 par adsorption dans les pores d'un support avec une solution d'adsorption contenant du rhénium et séchage entre 80°C et 170°C pendant 1 à 48 h, **caractérisé en ce que** seule la solution d'adsorption est chauffée à une température entre 40 et 80°C avant adsorption et on laisse la solution d'adsorption agir encore 1 à 30 minutes sur le support après l'adsorption.

7. Procédé de fabrication de catalyseurs selon l'une des revendications 1 à 5, **caractérisé en ce que** le support est placé dans un tambour d'adsorption rotatif, qui est mis en rotation entre 2 et 100 rotations par minute, arrosé avec une solution d'adsorption contenant du rhénium, moyennant quoi la quantité de solution correspond au volume poreux du support présenté, la quantité de solution arrose l'intérieur pendant 1 à 120 minutes, et on laisse la solution d'adsorption agir encore 1 à 30 minutes sur le support après l'adsorption, et le support imprégné est séché entre 80°C et 170°C pendant 1 à 48 h.

8. Utilisation de catalyseurs selon l'une des revendications 1 à 5 en tant que catalyseurs de métathèse.

9. Utilisation de catalyseurs selon l'une des revendications 1 à 5 pour la fabrication de propène par métathèse des oléfines en $C_4$.